(19) Europäisches Patentamt
European Patent Office
Office européen des brevets

(11) EP 0 760 664 B1

(12) EUROPÄISCHE PATENTSCHRIFT

(45) Veröffentlichungstag und Bekanntmachung des Hinweises auf die Patenterteilung:
**04.09.2002 Patentblatt 2002/36**

(21) Anmeldenummer: **95919422.6**

(22) Anmeldetag: **06.05.1995**

(51) Int Cl.[7]: **A61K 31/519**, A61P 43/00

(86) Internationale Anmeldenummer:
**PCT/EP95/01731**

(87) Internationale Veröffentlichungsnummer:
**WO 95/031987 (30.11.1995 Gazette 1995/51)**

(54) **VERWENDUNG VON PTERIDIN-DERIVATEN ALS HEMMSTOFFE DER NO-SYNTHASE**
USE OF PTERIDINE DERIVATIVES AS NO-SYNTHASE INHIBITORS
UTILISATION DE DERIVES DE PTERIDINE COMME INHIBITEURS DE LA NO-SYNTHASE

(84) Benannte Vertragsstaaten:
**AT BE CH DE DK ES FR GB GR IE IT LI LU MC NL PT SE**

(30) Priorität: **24.05.1994 DE 4418096**

(43) Veröffentlichungstag der Anmeldung:
**12.03.1997 Patentblatt 1997/11**

(73) Patentinhaber: **Vasopharm Biotech GmbH
97082 Würzburg (DE)**

(72) Erfinder:
- **PFLEIDERER, Wolfgang
D-78464 Konstanz (DE)**
- **SCHMIDT, Harald
D-97337 Dettelbach (DE)**
- **HENNING, Rainer
Shibuya-ku, Tokyo 151 (JP)**

(74) Vertreter: **Viering, Jentschura & Partner
Postfach 22 14 43
80504 München (DE)**

(56) Entgegenhaltungen:
**EP-A- 0 108 890** **CA-A- 2 075 346**

- **DATABASE WPI Section Ch, Week 9414 Derwent Publications Ltd., London, GB; Class B02, AN 94-111951 & JP-A-06 056 669 ( ASAHI BREWERIES LTD) , 1.März 1994**
- **BR. J. OF PHARMACOL., Bd. 106, Nr. 1, 1992 Seiten 222-226, NETZER T. ET AL 'Effects of a new pteridine derivative on urinary sodium, potassium and magnesium excretion in conscious saline-loaded rats'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 760 664 B1

Printed by Jouve, 75001 PARIS (FR)

## Beschreibung

Die vorliegende Erfindung betrifft Pteridin-Derivate der allgemeinen Formel I,

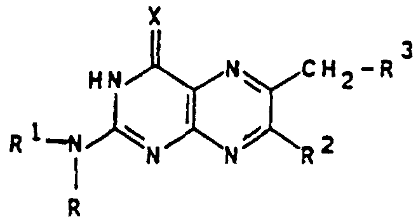

(I)

die aufgrund ihrer Fähigkeit zur Modulation der körpereigenen Stickstoffmonoxid-Produktion wertvolle Arzneimittel zur Vorbeugung und Bekämpfung von Krankheitszuständen sind, die durch einen gestörten Stickstoffmonoxid-Spiegel gekennzeichnet sind.

Stickstoffmonoxid (NO) spielt in verschiedensten physiologischen Prozessen eine bedeutende Rolle (siehe z.B. R.Henning, Nachr. Chem. Tech. Lab.41 (1993), 413; H.H.H.W.Schmidt et al., Biochim. Biophys. Acta 1178 (1993), 153). Es wirkt z.B. relaxierend auf die glatte Gefäßmuskulatur und ist auf diese Weise maßgeblich an der Regulierung des Blutdrucks beteiligt, es steuert über eine Hemmung der Thrombocytenaggregation die Blutgerinnung, und es ist beispielsweise im Gehirn als Neurotransmitter in den Aufbau des Langzeitgedächtnisses involviert. In den NANC-Nerven des peripheren Nervensystems fungiert NO ebenfalls als Botenstoff. Die zelltoxische Wirkung des NO wird von Macrophagen für die Infektionsabwehr ausgenutzt.

Endogenes NO wird mit Hilfe mindestens drei verschiedener NO-Synthase-Isoenzyme aus Arginin gebildet (siehe z.B. J.F.Kerwin Jr. und M.Heller, Med. Res. Rev. 14 (1994), 23). Sie unterscheiden sich bezüglich ihrer Lokalisation im Organismus, ihrer Regulierbarkeit durch $Ca^{2+}$/Calmodulin sowie ihrer Induzierbarkeit durch Endotoxine und Cytokine. Die konstitutiven, calciumabhängigen NO-Synthasen finden sich beispielsweise in Endothel (Typ III) und im Gehirn (Typ I) und sind dort in die Regulation von Blutdruck und -gerinnung bzw. in Reizleitungsprozesse involviert. Die cytokin-induzierbare, calciumunabhängige Isoform (Typ II) tritt in Macrophagen, Glattmuskelzellen und und Hepatocyten auf. Sie ist in der Lage, über einen langen Zeitraum relativ große Mengen NO zu produzieren und wird für entzündliche Prozesse und die zelltoxische Aktivität der Macrophagen verantwortlich gemacht.

Ein gestörter NO-Haushalt hat schwerwiegende Erkrankungen und Schädigungen zur Folge. So führt die exzessive Bildung von NO im septischen oder hämorrhagischen Schock zu massiven pathologischen Blutdruckabfällen. Übersteigerte NO-Produktion ist an der Entstehung von Typ-1-Diabetes und Atherosklerose beteiligt und scheint auch für die glutamatinduzierte Neurotoxizität nach cerebraler Ischämie verantwortlich zu sein. Hohe NO-Konzentrationen können darüberhinaus durch Desaminierung von Cytosin zu DNA-Schädigungen führen. Beispiele für Erkrankungen, die durch einen Mangel an endogenem NO mittelbar oder unmittelbar hervorgerufen werden, sind arterieller Bluthochdruck, Störungen der Hämostase, koronare Herzkrankheit und die erektile Dysfunktion.

Der Ansatz, eine Modulation der NO-Produktion zur Behandlung dieser Krankheitsbilder zu verwenden, wurde bislang nur mit Hilfes von Arginin-Analoga realisiert. (GB-A-2240041; WO-A-93/13055). Als weitere potentielle NO-Synthase-Hemmstoffe werden in der Literatur N-Iminoethylornithin (McCall et al, Br. J. Pharmacol.102 (1991), 234), Aminoguanidin (T.P.Misko et al, Eur. J. Pharmacol. 233 (1993), 119; EP-A-547588) und 7-Nitroindazol (P.K.Moore et al, Br.J.Pharmacol. 108 (1993), 296) diskutiert.

Verschiedene Pteridin-Derivate kommen in der Natur vor, und auch Verwendungen von Pteridin-Derivaten als Pharma-Wirkstoffe sind beschrieben. Das Zytostatikum Methotrexat ist ein Pteridin-Derivat. In der EP-B-290819 ist die Verwendung von Pteridinen, darunter auch solchen der allgemeinen Formel I, in denen $R^3$ für Hydroxy steht, für die Behandlung kognitiver Pathologien offenbart. Für Untersuchungen an der NO-Synthase, die sich mit mechanistischen Fragestellungen beschäftigten, wurden bislang vor allem hydrierte Pteridin-Derivate verwendet (siehe z.B. Kwon et al. (J. Biol. Chem. 264 (1989), 20496) oder Giovanelli et al. (Proc. Natl. Acad. Sci. USA 88 (1991), 7091)). Danach stimuliert Tetrahydrobiopterin die NO-Produktion und ist ein Cofaktor der NO-Synthasen. Eine Stimulation der NO-Produktion wurde auch für das 7,8-Dihydrobiopterin gefunden. Über eine Erhöhung der NO-Synthase-Aktivität durch 6-Methyl-5,6,7,8-tetrahydropterin berichten Hevel und Marletta (Biochemistry 31 (1992), 7160). Nicht hydrierte Pteridine, wie etwa Biopterin, Pterin, Folsäure oder 6-Hydroxymethylpterin zeigten in solchen Untersuchungen keine signifikaten Effekte (Kwon et al., J. Biol. Chem. 264 (1989), 20496).

Überraschend wurde nun gefunden, daß Pteridin-Derivate der allgemeinen Formel I insbesondere hemmend die endogene NO-Produktion modulieren und somit als Arzneimittel bei Krankheiten geeignet sind, die durch einen überhöhten NO-Spiegel charakterisiert sind.

Gegenstand der vorliegenden Erfindung ist die Verwendung von Pteridin-Derivaten der allgemeinen Formel I,

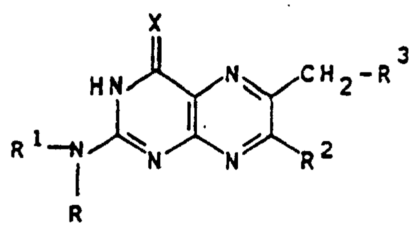

(I)

in der

X für O, NH oder N-($C_1$-$C_5$)-Alkanoyl steht;
R für Wasserstoff steht und
$R^1$ für Wasserstoff oder ($C_1$-$C_5$)-Alkanoyl steht oder R und $R^1$ zusammen mit dem Stickstoffatom, an das sie gebunden sind, eine Dimethylaminomethylenaminogruppe bilden;
$R^2$ für Wasserstoff, Methyl, Phenyl, Hydroxy, Methoxy oder Amino steht;
$R^3$ für den Rest -$OR^4$, -$NR^5R^6$ oder -$S(O)_mR^7$, wobei m für die Zahlen 0, 1 oder 2 steht, steht;
$R^4$ für Wasserstoff, ($C_1$-$C_{10}$)-Alkyl, Cyclohexyl, Benzyl, unsubstituiertes oder durch Chlor oder den Rest -$COR^8$ substituiertes Phenyl, unsubstituiertes oder am Stickstoff durch einen oder zwei gleiche oder verschiedene ($C_1$-$C_4$)-Alkylreste substituiertes Aminocarbonyimethyl, 2-Methoxyethyl, den (2,2-Dimethyl-1,3-dioxolan-4-yl) methyl-Rest oder den Rest -$COR^9$ steht;
$R^5$ für Wasserstoff, Methyl, Ethyl, 2-Hydroxyethyl, 2-Chlorethyl, Benzyl, Pyridylmethyl, Phenylethyl, Pyridylethyl oder Acetyl steht;
$R^6$ unabhängig von der Bedeutung von $R^5$ für die für $R^5$ angegebenen Bedeutungen steht oder, wenn $R^5$ für Wasserstoff oder Methyl steht, auch für Cyclohexyl, 3-(2-Ethoxyethoxy)propyl, Benzyl, das am Phenylring ein oder zwei Chloratome oder den Rest -$COR^{10}$ trägt, ($C_1$-$C_5$)-Alkanoyl, den Rest -$COR^{10}$ oder den Rest -$(CH_2)_4$-$COR^{10}$ steht;
$R^7$ für ($C_1$-$C_4$)-Alkyl, Benzyl, unsubstituiertes oder durch Chlor, den Rest -$COR^8$ oder den Rest -CO-O-CO--($C_1$-$C_4$) -Alkyl substituiertes Phenyl oder für Naphthyl steht;
$R^8$ für Hydroxy, Methoxy, Amino oder $R^{10}$ steht;
$R^9$ für ($C_1$-$C_4$)-Alkyl, Hydroxymethyl, Trifluormethyl, ($C_1$-$C_2$)-Alkoxy oder $R^{11}$ steht;
$R^{10}$ für den Rest

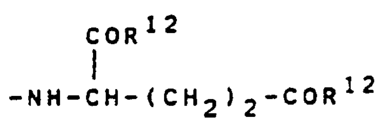

steht;
$R^{11}$ für den Rest

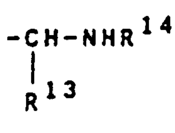

steht;
$R^{12}$ für Hydroxy oder ($C_1$-$C_2$)-Alkoxy steht;
$R^{13}$ für ($C_1$-$C_4$)-Alkyl oder Benzyl steht;
$R^{14}$ für Wasserstoff oder Benzyloxycarbonyl steht;

und ihrer tautomeren Formen sowie ihrer pharmakologisch verträglichen Salze zur Herstellung eines Arzneimittels zur

Vorbeugung und Behandlung von Krankheiten, die durch einen erhöhten Stickstoffmonoxid-Spiegel bedingt sind.

Alkylgruppen können geradkettig oder verzweigt sein. Dies gilt auch, wenn sie in anderen Gruppen, beispielsweise in Alkoxy-, Alkylmercapto-, Alkoxycarbonyl- oder Alkanoylgruppen auftreten. Beispiele für Alkylgruppen, die in den erfindungsgemäß zu verwendenden Verbindungen der allgemeinen Formel als solche, also als ($C_1$-$C_4$)- oder ($C_1$-$C_{10}$)-Alkyl, oder in anderen Gruppen auftreten können, sind Methyl, Ethyl, n-Propyl, i-Propyl-, n-Butyl, i-Butyl, sek-Butyl, tert-Butyl, n-Pentyl, 3-Methylbutyl, 2,2-Dimethylpropyl, n-Hexyl, n-Heptyl, n-Octyl, n-Nonyl oder n-Decyl. Beispiele speziell für ($C_1$-$C_5$)-Alkanoyl sind Formyl, Acetyl, Propionyl, n-Butyryl, i-Butyryl, n-Valeroyl, 3-Methyl-n-butyryl oder 2,2-Dimethylpropionyl, Beispiele für ($C_1$-$C_2$)-Alkoxy sind Methoxy und Ethoxy.

Ein Pyridylrest kann ein 2-Pyridyl-, 3-Pyridyl- oder 4-Pyridylrest sein, bevorzugt ist er ein 2-Pyridylrest. Ein Phenylrest, der einen Substituenten trägt, kann diesen in der 2-, der 3- oder der 4-Position tragen. Bevorzugt sind die 3- und die 4-Position, besonders bevorzugt ist die 4-Position. Trägt der Phenylrest zwei Substituenten, so können diese beispielsweise 2,3-, 2,4-, 3,4- oder 3,5-ständig sein. Bevorzugt sind sie 2,4- oder 3,4-ständig. Ein Naphthylrest kann ein 1-Naphthyl- oder 2-Naphthylrest sein, bevorzugt ist ein 2-Naphthylrest. In Phenylethylund Pyridylethylresten kann der Phenyl- bzw. Pyridylrest 1-ständig oder 2-ständig sein, bevorzugt ist er 2-ständig.

Die Verbindungen der allgemeinen Formel I können in verschiedenen tautomeren Formen und in verschiedenen stereoisomeren Formen vorliegen. Die vorliegende Erfindung umfaßt nicht nur die Verwendung aller tautomeren Formen, sondern auch die aller stereoisomeren Formen, also z.B. die von reinen Enantiomeren, von Enantiomerengemischen und Racematen, von reinen Diastereomeren und Diastereomerengemischen.

X steht bevorzugt für O oder NH.

Bevorzugt steht R für Wasserstoff.

$R^1$ steht bevorzugt für Wasserstoff.

$R^2$ steht bevorzugt für Wasserstoff. Steht $R^2$ für ($C_1$-$C_5$)-Alkanoyl, so sind Acetyl, i-Butyryl und Pivaloyl bevorzugt.

$R^3$ steht bevorzugt für ($C_1$-$C_{10}$)-Alkyloxy, Phenyloxy, Amino, Methylamino, Dimethylamino oder für den Rest -OCOR$^{11}$. Besonders bevorzugt steht $R^3$ für ($C_5$-$C_{10}$)-Alkyloxy, Amino oder den Rest -OCOR$^{11}$, wobei das in $R^{11}$ enthaltene $R^{14}$ für Benzyloxycarbonyl steht und das in $R^{11}$ enthaltene $R^{13}$ für Methyl, Isopropyl oder Benzyl steht.

Die Verbindungen der allgemeinen Formel I sind bekannt und können nach oder analog zu bekannten Verfahren hergestellt werden. Synthesemethoden für Pteridin-Derivate der allgemeinen Formel I sind z.B. die Methode von Gabriel-Isay oder die Taylor-Methode (siehe z.B. D.J.Brown, Fused Pyrimidines III, Pteridines (E.C. Taylor und A. Weissberger (Ed.), Wiley & Sons, New York)). Im einzelnen ist die Herstellung von Verbindungen der allgemeinen Formel I z.B. beschrieben in der EP-A-108 890, in der Dissertationsschrift von Hermann Michael Traub (Dissertation der Universität Konstanz, Deutschland (1987)), oder in J. Med. Chem. 30 (1987), 40.

Die erfindungsgemäß zu verwendenden Verbindungen der allgemeinen Formel I können mit anorganischen oder organischen Säuren Salze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Verbindungen der allgemeinen Formel I können ein oder mehrere Säureäquivalente addieren. Die Säureadditionssalze können wie üblich durch Vereinigung der Komponenten, zweckmäßigerweise in einem geeigneten Lösungs- oder Verdünnungsmittel, hergestellt werden. Durch Anionenaustausch können Säureadditionssalze ineinander überführt werden. Verbindungen der allgemeinen Formel I, die saure Gruppen enthalten, können mit anorganischen oder organischen Basen Salze bilden. Beispielsweise für solche Salze sind z.B. Alkalimetallsalze, insbesondere Natrium- und Kaliumsalze, oder Ammoniumsalze, insbesondere solche mit organischen Resten am Ammoniumstickstoff.

Die Hemmung der NO-Freisetzung durch die Erfindungen der allgemeinen Formel I kann durch einen Aktivitätsassay bestimmt werden, der auf Arbeiten von Bredt und Snyder sowie Schmidt et al. basiert (s. D.S.Bredt und S.S. Snyder, Isolation of nitric oxide synthase, a calmodulin-requiring enzyme, Proc.Natl.Acad.Sci. USA 87 (1990), 682; H.H.H.W.Schmidt et al., Purification of a soluble isoform of guanylyl cyclase-activating factor synthase, Proc. Natl. Acad.Sci. USA 88 (1991), 365). Hierbei wird für gereinigte NO-Synthase (NOS) das bei der NO-Bildung anfallende Coprodukt L-Citrullin quantitativ erfaßt. Dies geschieht durch den Einsatz von $^3$H-radiomarkiertem L-Arginin als Substrat der Enzymreaktion, das zu $^3$H-L-Citrullin und NO umgesetzt wird. Nach Beendigung der Enzyminkubation wird entstandenes L-Citrullin von unverbrauchtem L-Arginin mittels Ionenaustauschchromatographie aus dem Reaktionsgemisch entfernt; die durch Flüssigkeitsszintillationsmessung ermittelte $^3$H-Aktivität entspricht dann der Menge an L-Citrullin. Einzelheiten der Durchführung sind weiter unten angegeben.

Krankheiten, die durch einen erhöhten NO-Spiegel entstehen und die somit erfindungsgemäß mit den Verbindungen der allgemeinen Formel I behandelt werden können bzw. denen mit diesen vorgebeugt werden kann, sind insbesondere pathologische Blutdruckabfälle, wie sie beim septischen oder hämorrhagischen Schock, bei der Tumor- bzw. Krebstherapie mit Cytokinen oder bei der Leberzirrhose auftreten. Des weiteren entzündliche Erkrankungen, wie rheumatoide Arthritis und insbesondere Colitis ulcerosa, sowie Insulin-abhängiger Diabetes mellitus und Transplantat-Abstoßungsreaktionen.

Aber auch die folgenden Erkrankungen stehen im Zusammenhang mit einer gesteigerten Produktion von Stickstoffmonoxid und können erfindungsgemäß behandelt bzw. vorgebeugt werden. Im Bereich Herz-Kreislauf sind dies Arteriosklerose, postischämische Gewebeschäden und Infarktschäden, Reperfusionsschäden, Myokarditis auf der Grundlage einer Coxsackie-Virus-Infektion und Kardiomyopathie; im Bereich Nervensystem/Zentrales Nervensystem Neuritiden unterschiedlicher Ätiogenese (Neuritis formen), Enzephalomyelitiden, virale neurodegenerative Erkrankungen, Morbus Alzheimer, Hyperalgesie, Epilepsie und Migräne, wobei die Behandlung bzw. Vorbeugung des Morbus Alzheimer ausgenommen ist, wenn $R^3$ in der allgemeinen Formel I für Hydroxy steht; im Bereich Niere akutes Nierenversagen sowie Nephritiden unterschiedlicher Ätiogenese, speziell Glomerulonephritis.

Außerdem sind auch Behandlungen im Bereich des Magens und des Uterus/der Plazenta sowie eine Beeinflussung der Motilität der Spermien Anwendungsgebiete für die Verbindungen der allgemeinen Formel I.

Die Verbindungen der allgemeinen Formel I und ihre pharmakologisch annehmbaren Salze können als Hilfsstoffe in biochemischen und pharmakologischen Untersuchungen in der Forschung und in Diagnoseverfahren eingesetzt werden, und sie können am Tier, bevorzugt am Säugetier, und insbesondere am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen, enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen oder Infusionslösungen, oder perkutan, z.B. in Form von Salben oder Tinkturen, erfolgen.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Salze und noch andere therapeutisch wirksame Stoffe enthalten.

Derartige andere therapeutisch wirksame Substanzen sind beispielsweise: β-Rezeptorenblocker, wie z.B. Prppranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z. B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon; entzündungshemmende Substanzen, wie etwa Corticosteroide, Salicylate oder Propionsäurederivate, wie beispielsweise Ibuprofen; Antibiotika, wie z.B. Penicilline oder Cephalosporine; NO-Donoren, wie z.B. organische Nitrate, Sydnonimine oder Furoxane.

Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, in ähnlichen Mengenbereichen, d.h. im allgemeinen ebenfalls bei 0,5 bis 100 mg/Mensch. Die Tagesdosis kann in mehrere, z.B. 2 bis 4, Teilverabreichungen aufgeteilt werden.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Für verschiedene Pteridin-Derivate der allgemeinen Formel I sind bisher keine pharmakologischen Wirkungen oder medizinischen Verwendungen bekannt gewesen. Für solche Verbindungen gibt die vorliegende Erfindung die erste medizinische Indikation an. Gegenstand der vorliegenden Erfindung sind auch Pteridin-Derivate der allgemeinen Formel I,

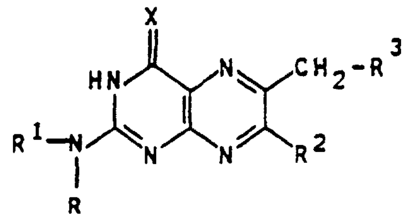

(I)

in der

X für O, NH oder N-($C_1$-$C_5$)-Alkanoyl steht;
R für Wasserstoff steht und
$R^1$ für Wasserstoff oder ($C_1$-$C_5$)-Alkanoyl steht oder R und $R^1$ zusammen mit dem Stickstoffatom, an das sie gebunden sind, eine Dimethylaminomethylenaminogruppe bilden;
$R^2$ für Wasserstoff, Methyl, Phenyl, Hydroxy, Methoxy oder Amino steht;
$R^3$ für den Rest -$OR^4$, -$NR^5R^6$ oder -$S(O)_mR^7$, wobei m für die Zahlen 0, 1 oder 2 steht, steht;
$R^4$ für Wasserstoff, ($C_1$-$C_{10}$)-Alkyl, Cyclohexyl, Benzyl, unsubstituiertes oder durch Chlor oder den Rest -$COR^8$ substituiertes Phenyl, unsubstituiertes oder am Stickstoff durch einen oder zwei gleiche oder verschiedene ($C_1$-$C_4$)-Alkylreste substituiertes Aminocarbonylmethyl, 2-Methoxyethyl, den (2,2-Dimethyl-1,3-dioxolan-4-yl) methyl-Rest oder den Rest -$COR^9$ steht;
$R^5$ für Wasserstoff, Methyl, Ethyl, 2-Hydroxyethyl, 2-Chlorethyl, Benzyl, Pyridylmethyl, Phenylethyl, Pyridylethyl oder Acetyl steht;
$R^6$ unabhängig von der Bedeutung von $R^5$ für die für $R^5$ angegebenen Bedeutungen steht oder, wenn $R^5$ für Wasserstoff oder Methyl steht, auch für Cyclohexyl, 3-(2-Ethoxyethoxy)propyl, Benzyl, das am Phenylring ein oder zwei Chloratome oder den Rest -$COR^{10}$ trägt, ($C_1$-$C_5$)-Alkanoyl, den Rest -$COR^{10}$ oder den Rest -$(CH_2)_4$-$COR^{10}$ steht;
$R^7$ für ($C_1$-$C_4$)-Alkyl, Benzyl, unsubstituiertes oder durch Chlor, den Rest -$COR^8$ oder den Rest -CO-O-CO-($C_1$-$C_4$) -Alkyl substituiertes Phenyl oder für Naphthyl steht;
$R^8$ für Hydroxy, Methoxy, Amino oder $R^{10}$ steht;
$R^9$ für ($C_1$-$C_4$)-Alkyl, Hydroxymethyl, Trifluormethyl, ($C_1$-$C_2$)-Alkoxy oder $R^{11}$ steht;
$R^{10}$ für den Rest

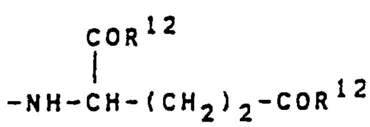

steht;
$R^{11}$ für den Rest

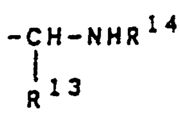

steht;
$R^{12}$ für Hydroxy oder ($C_1$-$C_2$)-Alkoxy steht;
$R^{13}$ für ($C_1$-$C_4$)-Alkyl oder Benzyl steht;
$R^{14}$ für Wasserstoff oder Benzyloxycarbonyl steht;

und ihrer tautomeren Formen sowie ihrer pharmakologisch verträglichen Salze, wobei aber Verbindungen der allgemeinen Formel I, in der $R^3$ für Hydroxy steht, ausgeschlossen sind und die Verbindung der allgemeinen Formel I, in der X für NH steht, R und $R^1$ für Wasserstoff stehen, $R^2$ für Amino steht und $R^3$ für Dimethylamino steht, ausgeschlossen ist, zur Verwendung als Arzneimittel. Für bevorzugte solche Pteridin-Derivate gilt das oben Gesagte entsprechend.

Die folgenden Beispiele geben Verbindungen der allgemeinen Formel I an, die erfindungsgemäß eingesetzt werden können. In den Beispielen werden folgende Abkürzungen verwendet:

Me = Methyl
Et = Ethyl
iPr = Isopropyl
iBu = Isobutyl
tBu = tert-Butyl
Ph = Phenyl
Py = 2-Pyridyl
Z = Benzyloxycarbonyl

Ph-4-COOH, Ph-4-Cl und entsprechende Angaben bedeuten einen Phenylrest, der in der 4-Position durch den Rest -COOH bzw. durch Chlor bzw. durch die im jeweiligen Beispiel angegebene Gruppe substituiert ist. $R^{10a}$, $R^{10b}$ und $R^{10c}$ stehen für den Rest der Formel

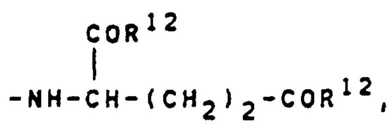

wobei im Fall $R^{10a}$ der Rest $R^{12}$ für Hydroxy, im Fall $R^{10b}$ der Rest $R^{12}$ für Ethoxy und im Fall $R^{10c}$ der Rest $R^{12}$ für Methoxy steht.

$R^a$ steht für den (2,2-Dimethyl-1,3-dioxolan-4-yl)methyl-Rest (D-Form).

In den Beispielen Nr. 1 bis 109 steht R in der allgemeinen Formel I für Wasserstoff.

| Nr. | X | $R^1$ | $R^2$ | $R^3$ |
|---|---|---|---|---|
| 1 | O | H | H | OH |
| 2 | O | tBuCO | H | OH |
| 3 | O | H | OH | OMe |
| 4 | O | H | H | OH |
| 5 | O | H | H | OEt |
| 6 | O | H | H | OiPr |
| 7 | O | H | H | OiBu |
| 8 | O | H | H | OtBu |
| 9 | O | H | H | OPh |
| 10 | O | H | H | O-(Ph-4-COOH) |
| 11 | O | H | H | O-(Ph-4-COOMe) |
| 12 | O | H | H | O-(Ph-4-$COR^{10a}$) |
| 13 | O | H | H | O-n-Octyl |
| 14 | O | H | H | O-n-Decyl |
| 15 | O | H | H | $OCH_2CH_2OMe$ |
| 16 | O | H | H | OCOMe |
| 17 | O | MeCO | H | OCOMe |
| 18 | O | H | H | OCOtBu |
| 19 | O | tBuCO | H | OCOtBu |
| 20 | O | H | H | $OCOCH_2OH$ |
| 21 | O | H | H | OCOCH(Me)-NHZ |
| 22 | O | H | H | OCOCH(iPr)-NHZ |
| 23 | O | H | H | $OCOCH(CH_2Ph)$-NHZ |
| 24 | O | H | H | $OCOCF_3$ |
| 25 | O | H | H | OCOOEt |
| 27 | O | H | H | NHMe |

(fortgesetzt)

| Nr. | X | $R^1$ | $R^2$ | $R^3$ |
|---|---|---|---|---|
| 28 | O | H | H | $NMe_2$ |
| 29 | O | H | H | $NH-CH_2-(Ph-4-COR^{10a})$ |
| 30 | O | H | H | $NH-CH_2-(Ph-4-COR^{10b})$ |
| 31 | O | tBuCO | H | $N(Me)-CH_2CH_2-Py$ |
| 32 | O | tBuCO | H | $N(CH_2CH_2-Py)_2$ |
| 33 | O | tBuCO | H | $N(CH_2-Py)_2$ |
| 34 | O | H | H | $N(CH_2CH_2-OH)_2$ |
| 35 | O | H | H | $N(CH_2CH_2-Cl)_2$ |
| 36 | O | H | H | $NH-COR^{10a}$ |
| 37 | O | H | H | SMe |
| 38 | O | H | H | SEt |
| 39 | O | H | H | S-n-Propyl |
| 49 | O | H | H | S-n-Butyl |
| 41 | O | H | H | S-(Ph-4-CO-O-COiBu) |
| 42 | O | H | H | S-(Ph-4-COOH) |
| 43 | O | H | H | S-(Ph-4-COOMe) |
| 44 | O | H | H | $S-(Ph-4-COR^{10a})$ |
| 45 | O | H | H | $S-(Ph-4-COR^{10b})$ |
| 46 | O | H | H | $S(O)_2Me$ |
| 47 | O | tBuCO | H | $S(O)_2Me$ |
| 48 | NH | H | H | OH |
| 49 | NH | H | Me | OH |
| 50 | NH | H | OMe | OH |
| 51 | NH | H | OH | OH |
| 52 | NH | H | H | OMe |
| 53 | NH | H | H | OEt |
| 54 | NH | H | H | O-n-Propyl |
| 55 | NH | H | H | OiPr |
| 56 | NH | H | H | O-n-Butyl |
| 57 | NH | H | H | OiBu |
| 58 | NH | H | H | OtBu |
| 59 | NH | H | H | O-n-Octyl |
| 60 | NH | H | H | O-n-Decyl |
| 61 | NH | H | H | $OCH_2CH_2OMe$ |
| 62 | NH | H | H | O-Cyclohexyl |
| 63 | NH | H | H | $O-CH_2Ph$ |
| 64 | NH | H | H | OPh |
| 65 | NH | H | H | O-(Ph-4-Cl) |
| 66 | NH | H | H | O-(Ph-4-COOH) |
| 67 | NH | H | H | $O-(Ph-4-CONH_2)$ |
| 68 | NH | H | H | $O-(Ph-4-COR^{10a})$ |
| 69 | NH | H | H | $O-(Ph-4-COR^{10b})$ |
| 70 | NH | H | H | $O-R^a$ |
| 71 | NH | H | H | OCOMe |
| 72 | NH | COMe | H | OCOMe |
| 73 | NH | H | H | $OCOCH_2OH$ |
| 74 | NH | H | H | OCOCH(Me)-NHZ |
| 75 | NH | H | H | $OCH_2CONEt_2$ |
| 76 | NH | H | H | $NH_2$ |

(fortgesetzt)

| Nr. | X | $R^1$ | $R^2$ | $R^3$ |
|---|---|---|---|---|
| 77 | NH | H | H | NHMe |
| 78 | NH | H | H | $NMe_2$ |
| 79 | NH | H | $NH_2$ | $NMe_2$ |
| 80 | NH | H | H | $NEt_2$ |
| 81 | NH | H | H | $NH-CH_2CH_2Ph$ |
| 82 | NH | H | H | $NH-CH_2(Ph-4-COR^{10a})$ |
| 83 | NH | H | H | $NH-CH_2(Ph-4-COR^{10b})$ |
| 84 | NH | H | H | $N(Me)-(CH_2)_4-COR^{10a}$ |
| 85 | NH | H | H | $N(Me)-(CH_2)_4-COR^{10b}$ |
| 86 | NH | H | H | $N(Me)-(CH_2)_4-COR^{10c}$ |
| 87 | NH | H | H | $NH-CH_2$-(2,4-Dichlorphenyl) |
| 88 | NH | H | H | $NH-CH_2$-(3,4-Dichlorphenyl) |
| 89 | NH | H | H | $NH-(CH_2)_3-O-(CH_2)_2-OEt$ |
| 90 | NH | H | H | NH-Cyclohexyl |
| 91 | NH | H | H | $(CH_2CH_2-OH)_2$ |
| 92 | NH | H | H | $N(CH_2CH_2-Cl)_2$ |
| 93 | NH | H | H | NH-COiPr |
| 94 | NH | H | H | SMe |
| 95 | NH | H | H | $SCH_2Ph$ |
| 96 | NH | H | H | SPh |
| 97 | NH | H | H | S-(Ph-4-COOH) |
| 98 | NH | H | H | S-(Ph-4-COOMe) |
| 99 | NH | H | H | S-(Ph-4-COOEt) |
| 100 | NH | H | H | S-(Ph-4-Cl) |
| 101 | NH | H | H | S(O)-(Ph-4-Cl) |
| 102 | NH | H | H | $S(O)_2$-(Ph-4-Cl) |
| 103 | NH | H | H | S-(Ph-4-CO-O-COiBu) |
| 104 | NH | H | H | $S-(Ph-4-COR^{10a})$ |
| 105 | NH | H | H | $S-(Ph-4-COR^{10b})$ |
| 106 | NH | H | H | S-(2-Naphthyl) |
| 107 | NCOMe | MeCO | H | OCOMe |
| 108 | NCOMe | MeCO | H | $N(COMe)_2$ |
| 109 | NCOiPr | iPrCO | H | NH-COiPr |

Beispiel 110

Verbindung der Formel

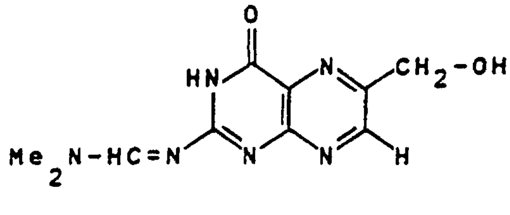

**Messung der Hemmung der Aktivität gereinigter Stickstoffmonoxid-Synthase (NOS)**

Bei diesem Aktivitätsassay wird das bei der Bildung von NO durch gereinigte NOS anfallende Koprodukt L-Citrullin quantitativ erfaßt. Als Substrat der Enzymreaktion wird $^3H$-radiomarkiertes L-Arginin eingesetzt, das

zu $^3$H-L-Citrullin und NO umgesetzt wird. Nach Beendigung der Enzyminkubation wird entstandenes L-Citrullin von unverbrauchtem L-Arginin mittels Ionenaustauschchromatographie aus dem Reaktionsgemisch entfernt; die durch Flüssigkeitsszintillation gemessene $^3$H-Aktivität entspricht dann der Menge an L-Citrullin, die ein direktes Maß für die Aktivität der NOS ist.

Das Grundmedium für die Durchführung der Enzymreaktion ist TE-Puffer (Triethanolamin, EDTA, pH 7,0). Das Endvolumen jeder Inkubation beträgt 100 µl. Das Reaktionsgemisch wird erhalten, indem die folgenden 6 Komponenten auf Eis gemischt werden:

1. "REA-Mix" (pH 7,0), der Triethanolamin, Calciumchlorid. Magnesiumchlorid, EDTA, L-Arginin, Calmodulin und Flavin-Adenin-Dinukleotid (FAD) enthält;
2. frisch zubereitete Stammlösung von β-Nicotinamid-Adenin-Dinukleotid-Phosphat, reduzierte Form (NADPH);
3. (6R)-5,6,7,8-Tetrahydro-L-Biopterin-Dihydrochlorid-Stammlösung ($BH_4$) oder - für Versuche ohne $BH_4$ - stattdessen TE-Puffer;
4. gereinigte NO-Synthase aus Schweinekleinhirn oder aus Schweineleber;
5. L-[2,3,4,5-$^3$H]-Arginin-Hydrochlorid-Stammlösung (1,5-2,6 TBq/mmol);
6. zu testende Substanz.

Die finalen Konzentrationen der Komponenten im Inkubationsvolumen von 100 µl sind: Triethanolamin 50 mM, EDTA 0,5 mM, $CaCl_2$ 226 µM, $MgCl_2$ 477 µM, L-Arginin 50 µM, Calmodulin 0,5 µM, FAD 5 µM, NADPH 1 mG, $BH_4$ (wenn zugesetzt) 2 µM, zu testende Substanz 100 µM. Nach dem Mischen der Komponenten auf Eis wird der Reaktionsansatz sofort in einem Wasserbad bei 37°C für 15 Minuten inkubiert. Nach dieser Inkubationszeit wird die Reaktion durch Zugabe von 900 µl eiskaltem "Stoppuffer" (20 mM Natriumacetat, 2 mM EDTA, pH 5,5) abgestoppt und der Ansatz (Gesamtvolumen jetzt 1,0 ml) auf Eis gestellt. Zur Abtrennung des nicht umgesetzten $^3$H-L-Arginins wird das Gemisch auf eine Ionenaustauschersäule mit 0,8 ml Dowex AG 50 WX-8 (100-200 mesh) gegeben, die zuvor mit 2 ml Stoppuffer gespült und äquilibriert wurde. Nach dem Auftragen der Probe wird die Säule zweimal mit je 1 ml Wasser eluiert. Der Durchlauf der Probe und das Eluat werden in Szintillationsgefäßen aufgefangen und gereinigt (Gesamtvolumen 3 ml). Zu den 3 ml wäßriger Meßlösung werden 9 ml Szintillator-Lösung gegeben und die homogene Mischung wird in einem Flüssigszintillationszähler Tricarb 2500 TR (Packard) 1 Minute pro Probe gemessen. Die mit der zu testenden Substanz gefundene Aktivität wird in Prozent der Aktivität der Kontrolle angegeben. Jede Substanz wird in einer Konzentration von 100 µM in Anwesenheit von 2 µM Tetrahydrobiopterin auf antagonistische Wirkung sowie in Abwesenheit von Tetrahydrobiopterin auf agonistische Wirkung auf die NOS getestet. Alle Inkubationen werden in Triplikaten angesetzt. Jeder Versuch wird dreimal mit verschiedenen Enzympräparationen wiederholt. Einige Ergebnisse sind in der folgenden Tabelle angegeben.

| Verbindung des Beispiels | Enzym aus | Citrullin-Bildung (% der Kontrolle) |
|---|---|---|
| 1 | Schweinehirn | 61,7 |
| 21 | Schweinehirn | 60,8 |
| 23 | Schweinehirn | 2,5 |
| 37 | Schweinehirn | 22,9 |
| 60 | Schweinehirn | 26,2 |
| 76 | Schweineleber | 22,4 |

**Beispiel A**

Gelatineweichkapseln, enthaltend 100 mg Wirkstoff pro Kapsel:

| | **pro Kapsel** |
|---|---|
| Wirkstoff | 100 mg |
| aus Kokosfett fraktioniertes Triclycerid-Gemisch | 400 mg |
| Kapselinhalt | 500 mg |

**Beispiel B**

Injektionslösung, enthaltend 2,0 mg Wirkstoff pro ml:

| | pro ml |
|---|---|
| Wirkstoff | 2,0 mg |
| Polyethylenglycol 400 | 5,0 mg |
| Natriumchlorid | 2,7 mg |
| Wasser zu Injektionszwecken | ad 1 ml |

**Beispiel C**

Emulsion, enthaltend 60 mg Wirkstoff pro 5 ml:

| | pro 100 ml Emulsion |
|---|---|
| Wirkstoff | 1,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

**Beispiel D**

Rektale Arzneiform, enthaltend 40 mg Wirkstoff pro Suppositorium:

| | pro Suppositorium |
|---|---|
| Wirkstoff | 40 mg |
| Suppositoriengrundmasse | ad 2 g |

**Beispiel E**

Tabletten, enthaltend 40 mg Wirkstoff pro Tablette:

| | pro Tablette |
|---|---|
| Wirkstoff | 40 mg |
| Lactose | 600 mg |
| Maisstärke | 300 mg |
| lösliche Stärke | 20 mg |
| Magnesiumstearat | 40 mg |
| | 1000 mg |

**Beispiel F**

Dragees, enthaltend 50 mg Wirkstoff pro Dragee:

| | pro Dragee |
|---|---|
| Wirkstoff | 50 mg |
| Maisstärke | 100 mg |
| Lactose | 60 mg |
| sec. Calciumphosphat | 30 mg |
| lösliche Stärke | 5 mg |
| Magnesiumstearat | 10 mg |
| kolloidale Kieselsäure | 5 mg |

(fortgesetzt)

| | pro Dragee |
|---|---|
| | 260 mg |

**Beispiel G**

Für die Herstellung des Inhalts von Hartgelatinekapseln eignen sich die folgenden Rezepturen:

| | | |
|---|---|---|
| a) | Wirkstoff | 100 mg |
| | Maisstärke | 300 mg |
| | | 400 mg |
| b) | Wirkstoff | 140 mg |
| | Milchzucker | 180 mg |
| | Maisstärke | 180 mg |
| | | 500 mg |

**Beispiel H**

Tropfen können nach folgender Rezeptur hergestellt werden (100 mg Wirkstoff in 1 ml = 20 Tropfen):

| | |
|---|---|
| Wirkstoff | 10 g |
| Benzoesäuremethylester | 0,07 g |
| Benzoesäureethylester | 0,03 g |
| Ethanol 96 %ig | 5 ml |
| entmineralisiertes Wasser | ad 100 ml |

**Patentansprüche**

**1.** Verwendung von Pteridin-Derivaten der allgemeinen Formel I,

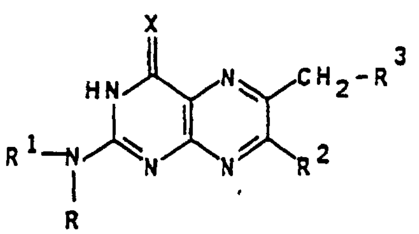

(I)

in der

X für O, NH oder N-($C_1$-$C_5$)-Alkanoyl steht;
R für Wasserstoff steht und
$R^1$ für Wasserstoff oder ($C_1$-$C_5$)-Alkanoyl steht oder R und $R^1$ zusammen mit dem Stickstoffatom, an das sie gebunden sind, eine Dimethylaminomethylenaminogruppe bilden;
$R^2$ für Wasserstoff, Methyl, Phenyl, Hydroxy, Methoxy oder Amino steht;
$R^3$ für den Rest -$OR^4$, -$NR^5R^6$ oder -$S(O)_mR^7$, wobei m für die Zahlen 0, 1 oder 2 steht, steht;
$R^4$ für Wasserstoff, ($C_1$-$C_{10}$)-Alkyl, Cyclohexyl, Benzyl, unsubstituiertes oder durch Chlor oder den Rest -$COR^8$ substituiertes Phenyl, unsubstituiertes oder am Stickstoff durch einen oder zwei gleiche oder verschiedene ($C_1$-$C_4$)-Alkylreste substituiertes Aminocarbonylmethyl, 2-Methoxyethyl, den (2,2-Dimethyl-1,3-dioxolan-4-yl)methyl-Rest oder den Rest -$COR^9$ steht;
$R^5$ für Wasserstoff, Methyl, Ethyl, 2-Hydroxyethyl, 2-Chlorethyl, Benzyl, Pyridylmethyl, Phenylethyl, Pyridyle-

thyl oder Acetyl steht;

$R^6$ unabhängig von der Bedeutung von $R^5$ für die für $R^5$ angegebenen Bedeutungen steht oder, wenn $R^5$ für Wasserstoff oder Methyl steht, auch für Cyclohexyl, 3-(2-Ethoxyethoxy)propyl, Benzyl, das am Phenylring ein oder zwei Chloratome oder den Rest $-COR^{10}$ trägt, ($C_1$-$C_5$-Alkanoyl, den Rest $-COR^{10}$ oder den Rest $-(CH_2)_4-COR^{10}$ steht;

$R^7$ für ($C_1$-$C_4$)-Alkyl, Benzyl, unsubstituiertes oder durch Chlor, den Rest $-COR^8$ oder den Rest -CO-O-CO-($C_1$-$C_4$)-Alkyl substituiertes Phenyl oder für Naphthyl steht;

$R^8$ für Hydroxy, Methoxy, Amino oder $R^{10}$ steht;

$R^9$ für ($C_1$-$C_4$)-Alkyl, Hydroxymethyl, Trifluormethyl, ($C_1$-$C_2$)-Alkoxy oder $R^{11}$ steht;

$R^{10}$ für den Rest

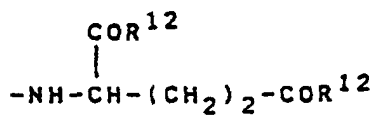

steht;

$R^{11}$ für den Rest

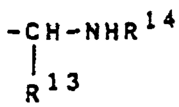

steht;

$R^{12}$ für Hydroxy oder ($C_1$-$C_2$)-Alkoxy steht;

$R^{13}$ für ($C_1$-$C_4$)-Alkyl oder Benzyl steht;

$R^{14}$ für Wasserstoff oder Benzyloxycarbonyl steht;

und ihrer tautomeren Formen sowie ihrer pharmakologisch verträglichen Salze zur Herstellung eines Arzneimittels zur Vorbeugung oder Behandlung von Krankheiten, die durch einen erhöhten Stickstoffmonoxid-Spiegel bedingt sind.

**2.** Verwendung gemäß Anspruch 1, **dadurch gekennzeichnet, daß** R für Wasserstoff steht und X für O oder NH steht.

**3.** Verwendung gemäß Anspruch 1 und/oder 2, **dadurch gekennzeichnet, daß** $R^1$ und/oder $R^2$ für Wasserstoff steht.

**4.** Verwendung gemäß einem oder mehreren der Ansprüche 1 bis 3, **dadurch gekennzeichnet, daß** $R^3$ für ($C_1$-$C_{10}$)-Alkyloxy, Phenyloxy, Amino, Methylamino, Dimethylamino oder den Rest $-OCOR^{11}$ steht, bevorzugt für ($C_5$-$C_{10}$)-Alkyloxy, Amino oder den Rest $-OCOR^{11}$, in dem das in $R^{11}$ enthaltene $R^{14}$ für Benzyloxycarbonyl und das in $R^{11}$ enthaltene $R^{13}$ für Methyl, Isopropyl oder Benzyl steht.

**5.** Verwendung gemäß einem oder mehreren der Ansprüche 1 bis 4 zur Herstellung eines Arzneimittels zur Behandlung oder Vorbeugung pathologischer Blutdruckabfälle, insbesondere beim septischen Schock oder der Tumortherapie mit Cytokinen.

**6.** Verwendung gemäß einem oder mehreren der Ansprüche 1 bis 4 zur Herstellung eines Arzneimittels zur Behandlung oder Vorbeugung von entzündlichen Erkrankungen, insbesondere Colitis ulcerosa.

**7.** Verwendung gemäß einem oder mehreren der Ansprüche 1 bis 4 zur Herstellung eines Arzneimittels zur Behandlung oder Vorbeugung von Infarktschäden und/oder Reperfusionsschäden.

**8.** Verwendung gemäß einem oder mehreren der Ansprüche 1 bis 4 zur Herstellung eines Arzneimittels zur Behandlung oder Vorbeugung von Transplantat-Abstoßungsreaktionen.

**9.** Verwendung gemäß einem oder mehreren der Ansprüche 1 bis 4 zur Herstellung eines Arzneimittels zur Behandlung oder Vorbeugung von Erkrankungen des Nervensystems aus der Reihe Morbus Alzheimer, Epilepsie und Migräne, wobei aber die Behandlung bzw. Vorbeugung des Morbus Alzheimer durch Verbindungen der allgemeinen Formel I ausgenommen ist, in der $R^3$ für Hydroxy steht.

**10.** Pteridin-Derivate der allgemeinen Formel I,

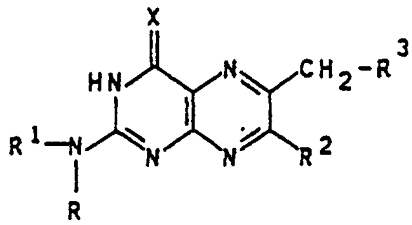

(I)

in der

X für O, NH oder N-($C_1$-$C_5$)-Alkanoyl steht;
R für Wasserstoff steht und
$R^1$ für Wasserstoff oder ($C_1$-$C_5$)-Alkanoyl steht oder R und $R^1$ zusammen mit dem Stickstoffatom, an das sie gebunden sind, eine Dimethylaminomethylenaminogruppe bilden;
$R^2$ für Wasserstoff, Methyl, Phenyl, Hydroxy, Methoxy oder Amino steht;
$R^3$ für den Rest -$OR^4$, -$NR^5R^6$ oder -$S(O)_mR^7$, wobei m für die Zahlen 0, 1 oder 2 steht, steht;
$R^4$ für Wasserstoff, ($C_1$-$C_{10}$)-Alkyl, Cyclohexyl, Benzyl, unsubstituiertes oder durch Chlor oder den Rest -$COR^8$ substituiertes Phenyl, unsubstituiertes oder am Stickstoff durch einen oder zwei gleiche oder verschiedene ($C_1$-$C_4$)-Alkylreste substituiertes Aminocarbonylmethyl, 2-Methoxyethyl, den (2,2-Dimethyl-1,3-dioxolan-4-yl)methyl-Rest oder den Rest -$COR^9$ steht;
$R^5$ für Wasserstoff, Methyl, Ethyl, 2-Hydroxyethyl, 2-Chlorethyl, Benzyl, Pyridylmethyl, Phenylethyl, Pyridylethyl oder Acetyl steht;
$R^6$ unabhängig von der Bedeutung von $R^5$ für die für $R^5$ angegebenen Bedeutungen steht oder, wenn $R^5$ für Wasserstoff oder Methyl steht, auch für Cyclohexyl, 3-(2-Ethoxyethoxy)propyl, Benzyl, das am Phenylring ein oder zwei Chloratome oder den Rest -$COR^{10}$ trägt, ($C_1$-$C_5$)-Alkanoyl, den Rest -$COR^{10}$ oder den Rest -$(CH_2)_4$-$COR^{10}$ steht;
$R^7$ für ($C_1$-$C_4$)-Alkyl, Benzyl; unsubstituiertes oder durch Chlor, den Rest -$COR^8$ oder den Rest -CO-O-CO-($C_1$-$C_4$)-Alkyl substituiertes Phenyl oder für Naphthyl steht;
$R^8$ für Hydroxy, Methoxy, Amino oder $R^{10}$ steht;
$R^9$ für ($C_1$-$C_4$)-Alkyl, Hydroxymethyl, Trifluormethyl, ($C_1$-$C_2$)-Alkoxy oder $R^{11}$ steht;
$R^{10}$ für den Rest

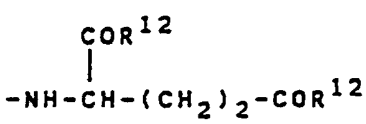

steht;
$R^{11}$ für den Rest

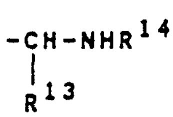

steht;

$R^{12}$ für Hydroxy oder $(C_1-C_2)$-Alkoxy steht;
$R^{13}$ für $(C_1-C_4)$-Alkyl oder Benzyl steht;
$R^{14}$ für Wasserstoff oder Benzyloxycarbonyl steht;

und ihrer tautomeren Formen sowie ihrer pharmakologisch verträglichen Salze, wobei aber Verbindungen der allgemeinen Formel I, in der $R^3$ für Hydroxy steht, ausgeschlossen sind und die Verbindung der allgemeinen Formel I, in der X für NH steht, R und $R^1$ für Wasserstoff stehen, $R^2$ für Amino steht und $R^3$ für Dimethylamino steht, ausgeschlossen ist, zur Verwendung als Arzneimittel.

**11.** Verfahren zur Herstellung eines Arzneimittels zur Vorbeugung oder Behandlung von Krankheiten, die durch einen erhöhten Stickstoffmonoxid-Spiegel bedingt sind, **dadurch gekennzeichnet, daß** als Wirkstoff mindestens ein Pteridin-Derivat der allgemeinen Formel I,

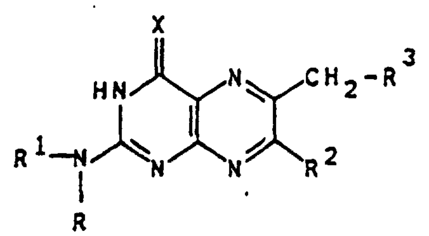

(I)

in der

X für O, NH oder N-$(C_1-C_5)$-Alkanoyl steht;
R für Wasserstoff steht und
$R^1$ für Wasserstoff oder $(C_1-C_5)$-Alkanoyl steht oder R und $R^1$ zusammen mit dem Stickstoffatom, an das sie gebunden sind, eine Dimethylaminomethylenaminogruppe bilden;
$R^2$ für Wasserstoff, Methyl, Phenyl, Hydroxy, Methoxy oder Amino steht;
$R^3$ für den Rest $-OR^4$, $-NR^5R^6$ oder $-S(O)_mR^7$, wobei m für die Zahlen 0, 1 oder 2 steht, steht;
$R^4$ für Wasserstoff, $(C_1-C_{10})$-Alkyl, Cyclohexyl, Benzyl, unsubstituiertes oder durch Chlor oder den Rest $-COR^8$ substituiertes Phenyl, unsubstituiertes oder am Stickstoff durch einen oder zwei gleiche oder verschiedene $(C_1-C_4)$-Alkylreste substituiertes Aminocarbonylmethyl, 2-Methoxyethyl, den (2,2-Dimethyl-1,3-dioxolan-4-yl)methyl-Rest oder den Rest $-COR^9$ steht;
$R^5$ für Wasserstoff, Methyl, Ethyl, 2-Hydroxyethyl, 2-Chlorethyl, Benzyl, Pyridylmethyl, Phenylethyl, Pyridylethyl oder Acetyl steht;
$R^6$ unabhängig von der Bedeutung von $R^5$ für die für $R^5$ angegebenen Bedeutungen steht oder, wenn $R^5$ für Wasserstoff oder Methyl steht, auch für Cyclohexyl, 3-(2-Ethoxyethoxy)propyl, Benzyl, das am Phenylring ein oder zwei Chloratome oder den Rest $-COR^{10}$ trägt, $(C_1-C_5)$-Alkanoyl, den Rest $-COR^{10}$ oder den Rest $-(CH_2)_4-COR^{10}$ steht;
$R^7$ für $(C_1-C_4)$-Alkyl, Benzyl, unsubstituiertes oder durch Chlor, den Rest $-COR^8$ oder den Rest $-CO-O-CO-(C_1-C_4)$-Alkyl substituiertes Phenyl oder für Naphthyl steht;
$R^8$ für Hydroxy, Methoxy, Amino oder $R^{10}$ steht;
$R^9$ für $(C_1-C_4)$-Alkyl, Hydroxymethyl, Trifluormethyl, $(C_1-C_2)$-Alkoxy oder $R^{11}$ steht;
$R^{10}$ für den Rest

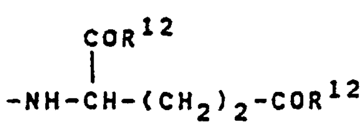

steht;
$R^{11}$ für den Rest

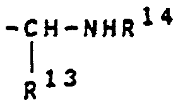

steht;

$R^{12}$ für Hydroxy oder ($C_1$-$C_2$)-Alkoxy steht;

$R^{13}$ für ($C_1$-$C_4$)-Alkyl oder Benzyl steht;

$R^{14}$ für Wasserstoff oder Benzyloxycarbonyl steht;

oder eine tautomere Form oder ein pharmakologisch verträgliches Salz davon verwendet wird.

## Claims

**1.** The use of pteridine derivates of the formula I

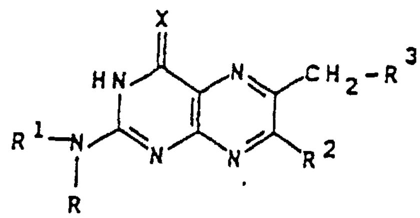

(I)

in which

X is O, NH or N-($C_1$-$C_5$)-alkanoyl;

R is hydrogen and

$R^1$ is hydrogen or ($C_1$-$C_5$)-alkanoyl or R and $R^1$ together with the nitrogen atom to which they are bonded form a dimethylaminomethyleneamino group;

$R^2$ is hydrogen, methyl, phenyl, hydroxyl, methoxy or amino;

$R^3$ is the radical $-OR^4$, $-NR^5R^6$ or $-S(O)_mR^7$, where m is the numbers 0, 1 or 2;

$R^4$ is hydrogen, ($C_1$-$C_{10}$)-alkyl, cyclohexyl, benzyl, phenyl which is unsubstituted or substituted by chlorine or the radical $-COR^8$, aminocarbonylmethyl which is unsubstituted or substituted on the nitrogen by one or two identical or different ($C_1$-$C_4$)-alkyl radicals, 2-methoxyethyl, the (2,2-dimethyl-1,3-dioxolan-4-yl) methyl radical or the radical $-COR^9$;

$R^5$ is hydrogen, methyl, ethyl, 2-hydroxyethyl, 2-chloroethyl, benzyl, pyridylmethyl, phenylethyl, pyridylethyl or acetyl;

$R^6$ independently of the meaning of $R^5$ has the meanings indicated for $R^5$ or, if $R^5$ is hydrogen or methyl, is also cyclohexyl, 3-(2-ethoxyethoxy)-propyl, benzyl which carries one or two chlorine atoms or the radical $-COR^{10}$ on the phenyl ring, ($C_1$-$C_5$)-alkanoyl, the radical $-COR^{10}$ or the radical $-(CH_2)_4-COR^{10}$;

$R^7$ is ($C_1$-$C_4$)-alkyl, benzyl, phenyl which is unsubstituted or substituted by chlorine, the radical $-COR^8$ or the radical -CO-O-CO-($C_1$-$C_4$)-alkyl or naphthyl;

$R^8$ is hydroxyl, methoxy, amino or $R^{10}$;

$R^9$ is ($C_1$-$C_4$)-alkyl, hydroxymethyl, trifluoromethyl, ($C_1$-$C_2$)-alkoxy or $R^{11}$;

$R^{10}$ is the radical

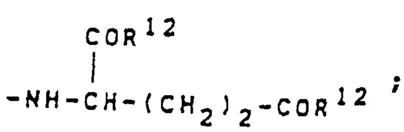

$R^{11}$ is the radical

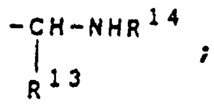

$R^{12}$ is hydroxyl or $(C_1-C_2)$-alkoxy;
$R^{13}$ is $(C_1-C_4)$-alkyl or benzyl;
$R^{14}$ is hydrogen or benzyloxycarbonyl;

and their tautomeric forms and their pharmacologically tolerable salts for the manufacture of a medicament for the prevention and treatment of diseases which are caused by an increased nitric oxide level.

**2.** The use as claimed in claim 1, wherein R is hydrogen and X is O or NH.

**3.** The use as claimed in claim 1 and/or 2, wherein $R^1$ and/or $R^2$ is hydrogen

**4.** The use as claimed in one or more of claims 1 to 3, wherein $R^3$ is $(C_1-C_{10})$-alkyloxy, phenyloxy, amino, methylamino, dimethylamino or the radical $-COR^{11}$, preferably $(C_5-C_{10})$-alkyloxy, amino or the radical $-COR^{11}$, in which the $R^{14}$ contained in $R^{11}$ is benzyloxycarbonyl and the $R^{13}$ contained in $R^{11}$ is methyl, isopropyl or benzyl.

**5.** The use as claimed in one or more of claims 1 to 4 for the manufacture of a medicament for the treatment and prevention of pathological blood pressure decreases, in particular in septic shock and tumor therapy with cytokines.

**6.** The use as claimed in one or more of claims 1 to 4 for the manufacture of a medicament for the treatment and prevention of inflammatory disorders, in particular ulcerative colitis.

**7.** The use as claimed in one or more of claims 1 to 4 for the manufacture of a medicament for the treatment and prevention of infarct damage and/or reperfusion damage.

**8.** The use as claimed in one or more of claims 1 to 4 for the manufacture of a medicament for the treatment and prevention of transplant rejection reactions.

**9.** The use as claimed in one or more of claims 1 to 4 for the manufacture of a medicament for the treatment and prevention of disorders of the nervous system selected from the group consisting of Alzheimer's disease, epilepsy and migraine, but where the treatment or prevention of Alzheimer's disease by compounds of the formula I in which $R^3$ is hydroxyl is excluded.

**10.** A pteridine derivative of the formula I

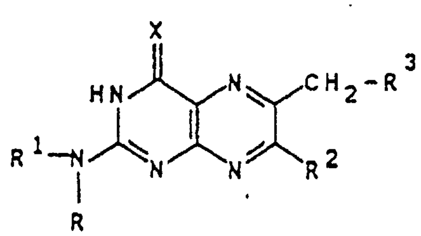

(I)

in which

X is O, NH or N-$(C_1-C_5)$-alkanoyl;
R is hydrogen and
$R^1$ is hydrogen or $(C_1-C_5)$-alkanoyl or R and $R^1$ together with the nitrogen atom to which they are bonded form a dimethylaminomethyleneamino group;
$R^2$ is hydrogen, methyl, phenyl, hydroxyl, methoxy or amino;
$R^3$ is the radical $-OR^4$, $-NR^5R^6$ or $-S(O)_mR^7$, where m is the numbers 0, 1 or 2;
$R^4$ is hydrogen, $(C_1-C_{10})$-alkyl, cyclohexyl, benzyl, phenyl which is unsubstituted or substituted by chlorine or

the radical $-COR^8$, aminocarbonylmethyl which is unsubstituted or substituted on the nitrogen by one or two identical or different $(C_1-C_4)$-alkyl radicals, 2-methoxyethyl, the (2,2-dimethyl-1,3-dioxolan-4-yl)methyl radical or the radical $-COR^9$;

$R^5$ is hydrogen, methyl, ethyl, 2-hydroxyethyl, 2-chloroethyl, benzyl, pyridylmethyl, phenylethyl, pyridylethyl or acetyl;

$R^6$ independently of the meaning of $R^5$ has the meanings indicated for $R^5$ or, if $R^5$ is hydrogen or methyl, is also cyclohexyl, 3-(2-ethoxyethoxy)-propyl, benzyl which carries one or two chlorine atoms or the radical $-COR^{10}$ on the phenyl ring, $(C_1-C_5)$-alkanoyl, the radical $-COR^{10}$ or the radical-$(CH_2)_4$-$COR^{10}$;

$R^7$ is $(C_1-C_4)$-alkyl, benzyl, phenyl which is unsubstituted or substituted by chlorine, the radical $-COR^8$ or the radical -CO-O-CO-$(C_1-C_4)$-alkyl or naphtyl;

$R^8$ is hydroxyl, methoxy, amino or $R^{10}$;

$R^9$ is $(C_1-C_4)$-alkyl, hydroxymethyl, trifluoromethyl, $(C_1-C_2)$-alkoxy or $R^{11}$;

$R^{10}$ is the radical

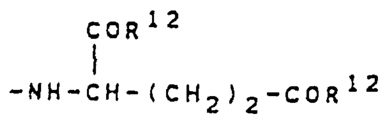 ;

$R^{11}$ is the radical

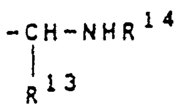 ;

$R^{12}$ is hydroxyl or $(C_1-C_2)$-alkoxy;

$R^{13}$ is $(C_1-C_4)$-alkyl or benzyl;

$R^{14}$ is hydrogen or benzyloxycarbonyl; and its tautomeric forms and also its pharmacologically tolerable salts where, however, compounds of the formula I in which $R^3$ is hydroxyl are excluded and the compound of the general formula I, in which X is NM, R and $R^1$ are hydrogen, $R^2$ is amino and $R^3$ is dimethylamino for the use as a medicament.

**11.** A method for preparation of a medicament for the prevention or treatment of diseases which are caused by an increased nitric oxide level, **characterised in that** at least one pteridine derivative of the general formula I or a tautomeric form or a pharmacologically tolerable salt thereof is used,

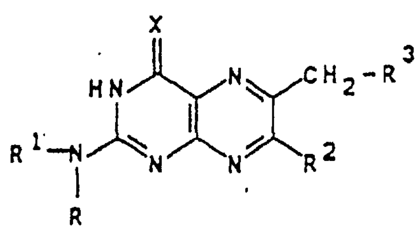

(I)

in which

X is O, NH or N-$(C_1-C_5)$-alkanoyl;

R is hydrogen and

$R^1$ is hydrogen or $(C_1-C_5)$-alkanoyl or R and $R^1$ together with the nitrogen atom to which they are bonded form a dimethylaminomethyleneamino group;

$R^2$ is hydrogen, methyl, phenyl, hydroxyl, methoxy or amino;

$R^3$ is the radical $-OR^4$, $-NR^5R^6$ or $-S(O)_mR^7$, where m is the numbers 0, 1 or 2;

$R^4$ is hydrogen, $(C_1-C_{10})$-alkyl, cyclohexyl, benzyl, phenyl which is unsubstituted or substituted by chlorine or the radical $-COR^8$, aminocarbonylmethyl which is unsubstituted or substituted on the nitrogen by one or two identical or different $(C_1-C_4)$-alkyl radicals, 2-methoxyethyl, the (2,2-dimethyl-1,3-dioxolan-4-yl)methyl radical or the radical $-COR^9$-;

$R^5$ is hydrogen, methyl, ethyl, 2-hydroxyethyl, 2-chloroethyl, benzyl, pyridylmethyl, phenylethyl, pyridylethyl or acetyl;

$R^6$ independently of the meaning of $R^5$ has the meanings indicated for $R^5$ or, if $R^5$ is hydrogen or methyl, is also cyclohexyl, 3-(2-ethoxyethoxy)-propyl, benzyl which carries one or two chlorine atoms or the radical $-COR^{10}$ on the phenyl ring, $(C_1-C_5)$-alkanoyl, the radical $-COR^{10}$ or the radical $-(CH_2)_4-COR^{10}$ ;

$R^7$ is $(C_1-C_4)$-alkyl, benzyl, phenyl which is unsubstituted or substituted by chlorine, the radical $-COR^8$ or the radical $-CO-O-CO-(C_1-C_4)$-alkyl or naphtyl;

$R^8$ is hydroxyl, methoxy, amino or $R^{10}$;

$R^9$ is $(C_1-C_4)$-alkyl, hydroxymethyl, trifluoromethyl, $(C_1-C_2)$-alkoxy or $R^{11}$;

$R^{10}$ is the radical

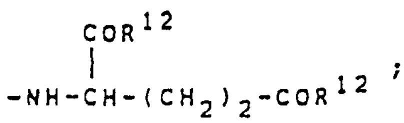

$R^{11}$ is the radical

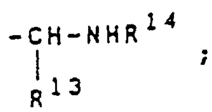

$R^{12}$ is hydroxyl or $(C_1-C_2)$-alkoxy;

$R^{13}$ is $(C_1-C_4)$-alkyl or benzyl;

$R^{14}$ is hydrogen or benzyloxycarbonyl; and its tautomeric forms and also its pharmacologically tolerable salts where, however, compounds of the formula I in which $R^3$ is hydroxyl are excluded and the compound of the general formula I, in which X is NM, R and $R^1$ are hydrogen, $R^2$ is amino and $R^3$ is dimethylamino for the use as a medicament.

**Revendications**

**1.** Utilisation de dérivés de ptéridine de formule générale I,

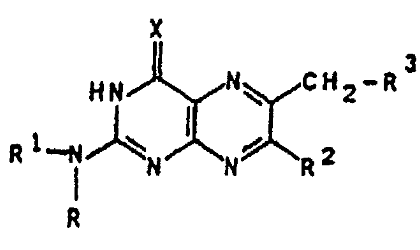

(I)

dans laquelle

X représente O, NH ou N-alcanoyle en $C_1$-$C_5$ ;

R représente l'hydrogène et

$R^1$ représente l'hydrogène ou un alcanoyle en $C_1$-$C_5$ ou bien R et $R^1$, pris ensemble avec l'atome d'azote auquel ils sont liés, forment un groupe diméthylaminométhylèneamino ;

$R^2$ représente l'hydrogène, un méthyle, un phényle, un hydroxy, un méthoxy ou un amino ;

$R^3$ représente un résidu $-OR^4$, $-NR^5R^6$ ou $-S(O)_mR^7$, où m représente le nombre 0, 1 ou 2 ;

$R^4$ représente l'hydrogène, ou un alkyle en $C_1$-$C_{10}$, cyclohexyle, benzyle, phényle non substitué ou substitué par un chlore ou un résidu $-COR^8$, aminocarbonylméthyle non substitué ou substitué par un ou plusieurs restes alkyle en $C_1$-$C_4$ identiques ou différents sur l'atome d'azote, 2-méthoxyéthyle, le reste (2,2-diméthyl-1,3-dioxolan-4-yl)méthyle ou le reste $-COR^9$ ;

$R^5$ représente l'hydrogène, le méthyle, l'éthyle, le 2-hydroxyéthyle, le 2-chloroéthyle, le benzyle, le pyridylméthyle, le phényléthyle, le pyridyléthyle ou l'acétyle ;

$R^6$ indépendamment de la signification de $R^5$, a la même signification que $R^5$, ou bien, si $R^5$ représente un hydrogène ou un méthyle, représente un cyclohexyle, un 3-(2-éthoxyéthoxy)propyle, un benzyle qui porte sur le cycle phényle un ou deux atomes de chlore ou bien le reste $-COR^{10}$, un alcanoyle en $C_1$-$C_5$, le reste $-COR^{10}$ ou le reste $-(CH_2)_4-COR^{10}$ ;

$R^7$ représente un alkyle en $C_1$-$C_4$, un benzyle, un phényle non substitué ou substitué par du chlore, le reste $-COR^8$ ou le reste -CO-O-CO-alkyle en $C_1$-$C_4$, ou un naphtyle ;

$R^8$ représente un groupe hydroxy, méthoxy, amino ou $R^{10}$ ;

$R^9$ représente un alkyle en $C_1$-$C_4$, hydroxyméthyle, trifluorométhyle, alcoxy en $C_1$-$C_2$ ou $R^{11}$ ;

$R^{10}$ représente le reste

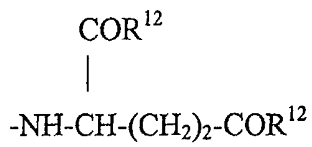
;

$R^{11}$ représente le reste

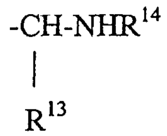
;

$R^{12}$ représente un hydroxy ou un alcoxy en $C_1$-$C_2$ ;

$R^{13}$ représente un groupe alkyle en $C_1$-$C_4$ ou benzyle ;

$R^{14}$ représente l'hydrogène ou un groupe benzyloxycarbonyle ;

et leurs formes tautomères ainsi que leurs sels pharmacologiquement acceptables, pour la fabrication d'un médicament pour la prévention et le traitement de maladies qui sont liées à une élévation du bilan du monoxyde de carbone.

2. Utilisation selon la revendication 1, **caractérisée en ce que** R représente l'hydrogène et X représente O ou NH.

3. Utilisation selon la revendication 1 et/ou 2, **caractérisée en ce que** $R^1$ et/ou $R^2$ représentent l'hydrogène.

4. Utilisation selon l'une quelconque des revendications 1 à 3, **caractérisée en ce que** $R^3$ représente un alkyloxy en $C_1$-$C_{10}$, phényloxy, amino, méthylamino, diméthylamino ou le reste $-OCOR^{11}$, de préférence le reste alcoxy en $C_5$-$C_{10}$, amino ou le reste $-OCOR^{11}$, dans lequel $R^{14}$ compris dans $R^{11}$ représente un benzyloxycarbonyle et le $R^{13}$ renfermé dans $R^{11}$ représente un méthyle, un isopropyle ou un benzyle.

5. Utilisation selon une ou plusieurs des revendications 1 à 4, pour la préparation d'un médicament pour le traitement ou la prévention de chutes de pression sanguine pathologiques, en particulier en cas de choc septicémique ou de thérapie tumorale à la cytokine.

6. Utilisation selon l'une quelconque des revendications 1 à 4 pour la préparation d'un médicament pour le traitement ou la prévention des maladies inflammatoires, en particulier la colitis ulcerosa.

7. Utilisation selon l'une ou plusieurs des revendications 1 à 4 pour la préparation d'un médicament pour le traitement ou la prévention des lésions d'infarctus et/ou des lésions de reperfusion.

**8.** Utilisation selon l'une ou plusieurs des revendications 1 à 4 pour la préparation d'un médicament pour le traitement ou la prévention des réactions de rejet de transplants.

**9.** Utilisation selon l'une ou plusieurs des revendications 1 à 4 pour la préparation d'un médicament pour le traitement ou la prévention de maladies du système nerveux de la famille de la maladie d'Alzheimer, épilepsie et migraine, mais dans lequel pour le traitement ou la prévention de la maladie d'Alzheimer à l'aide de composés de formule générale I, $R^3$ représenté un hydroxy.

**10.** Dérivés de ptéridine de formule générale I,

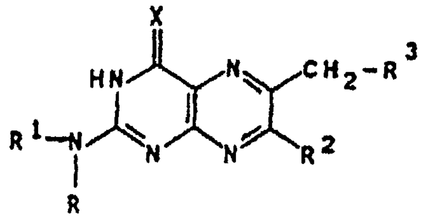

(I)

dans laquelle

X représente O, NH ou N-alcanoyle en $C_1$-$C_5$ ;
R représente l'hydrogène et
$R^1$ représente l'hydrogène ou un alcanoyle en $C_1$-$C_5$ ou bien R et $R^1$ forment ensemble avec l'atome d'azote auquel ils sont liés un groupe diméthylaminométhylèneamino ;
$R^2$ représente l'hydrogène, un méthyle, un phényle, un hydroxy, un méthoxy ou un amino ;
$R^3$ représente un reste $-OR^4$, $-NR^5R^6$ ou $-S(O)_mR^7$, où m représente le nombre 0, 1 ou 2;
$R^4$ représente l'hydrogène, un alkyle en $C_1$-$C_{10}$, un cyclohexyle, un benzyle, un phényle non substitué ou substitué par un chlore ou un reste $-COR^8$, aminocarbonylméthyle non substitué ou substitué sur l'azote par un ou deux restes alkyle en $C_1$-$C_4$ identiques ou différents, un 2-méthoxyéthyle, le reste (2,2-diméthyl-1,3-dioxolan-4-yl)méthyle ou le reste $-COR^9$ ;
$R^5$ représente l'hydrogène, un méthyle, un éthyle, un 2-hydroxyéthyle, un 2-chloroéthyle, un benzyle, un pyridylméthyle, un phényléthyle, un pyridyléthyle ou un acétyle ;
$R^6$ indépendamment de la signification de $R^5$, a la même signification que $R^5$, ou bien, si $R^5$ est un hydrogène ou un méthyle, il représente aussi un cyclohexyle, un 3-(2-éthoxyéthoxy)propyle, un benzyle qui porte sur le cycle phényle un ou deux atomes de chlore ou le reste $-COR^{10}$, un alcanoyle en $C_1$-$C_5$, le reste $-COR^{10}$ ou le reste $-(CH_2)_4-COR^{10}$ ;
$R^7$ représente un groupe alkyle en $C_1$-$C_4$, un benzyle, un phényle non substitué ou substitué par le chlore, le reste $-COR^8$ ou un reste -CO-O-CO-alkyle en $C_1$-$C_4$ ou un naphtyle ;
$R^8$ représente un hydroxy, un méthoxy, un amino ou $R^{10}$ ;
$R^9$ représente un alkyle en $C_1$-$C_4$, un hydroxyméthyle, un trifluorométhyle, un alcoxy en $C_1$-$C_2$ ou $R^{11}$ ;
$R^{10}$ représente le reste

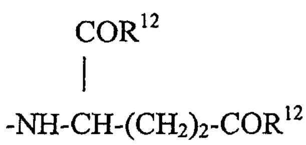

;

$R^{11}$ représente le reste

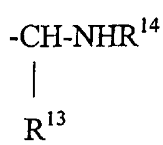

;

$R^{12}$ représente un hydroxy ou un alcoxy en $C_1$-$C_2$ ;

$R^{13}$ représente un alkyle en $C_1$-$C_4$ ou un benzyle ;
$R^{14}$ représente un hydrogène ou un benzyloxycarbonyle ;

et leurs formes tautomères ainsi que ses sels pharmacologiquement acceptables, mais où les composés de formule générale I, où $R^3$ représente un hydroxy, sont exclus et les composés de formule générale I, où X représente NH, R et $R^1$ représentent l'hydrogène, $R^2$ représente un amino et $R^3$ représente un diméthylamino, sont exclus, pour servir en tant que médicaments.

**11.** Procédé de préparation d'un médicament pour la prévention ou le traitement des maladies qui sont liées à un bilan trop élevé du monoxyde d'azote, **caractérisé en ce qu'**on utilise comme produit actif au moins un dérivé de ptéridine de formule générale I,

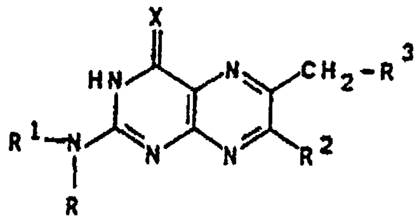

(I)

dans laquelle

X représente O, NH ou N-alcanoyle en $C_1$-$C_5$ ;
R représente l'hydrogène et
$R^1$ représente l'hydrogène ou un alcanoyle en $C_1$-$C_5$ ou bien R et $R^1$, pris ensemble avec l'atome d'azote auquel ils sont liés, forment un groupe diméthylaminométhylèneamino ;
$R^2$ représente l'hydrogène, un méthyle, un phényle, un hydroxy, un méthoxy ou un amino ;
$R^3$ représente un résidu $-OR^4$, $-NR^5R^6$ ou $-S(O)_mR^7$, où m représente le nombre 0, 1 ou 2;
$R^4$ représente l'hydrogène, ou un alkyle en $C_1$-$C_{10}$, cyclohexyle, benzyle, phényle non substitué ou substitué par un chlore ou un résidu $-COR^8$, aminocarbonylméthyle non substitué ou substitué par un ou plusieurs restes alkyle en $C_1$-$C_4$ identiques ou différents sur l'atome d'azote, 2-méthoxyéthyle, le reste (2,2-diméthyl-1,3-dioxolan-4-yl)méthyle ou le reste $-COR^9$ ;
$R^5$ représente l'hydrogène, le méthyle, l'éthyle, le 2-hydroxyéthyle, le 2-chloroéthyle, le benzyle, le pyridylméthyle, le phényléthyle, le pyridyléthyle ou l'acétyle ;
$R^6$ indépendamment de la signification de $R^5$, a la même signification que $R^5$, ou bien, si $R^5$ représente un hydrogène ou un méthyle, représente un cyclohexyle, un 3-(2-éthoxyéthoxy)propyle, un benzyle qui porte sur le cycle phényle un ou deux atomes de chlore ou bien le reste $-COR^{10}$, un alcanoyle en $C_1$-$C_5$, le reste $-COR^{10}$ ou le reste $-(CH_2)_4-COR^{10}$ ;
$R^7$ représente un alkyle en $C_1$-$C_4$, un benzyle, un phényle non substitué ou substitué par du chlore, le reste $-COR^8$ ou le reste -CO-O-CO-alkyle en $C_1$-$C_4$, ou un naphtyle ;
$R^8$ représente un groupe hydroxy, méthoxy, amino ou $R^{10}$ ;
$R^9$ représente un alkyle en $C_1$-$C_4$, hydroxyméthyle, trifluorométhyle, alcoxy en $C_1$-$C_2$ ou $R^{11}$ ;
$R^{10}$ représente le reste

$$\begin{array}{l} \quad\quad\;\; COR^{12} \\ \quad\quad\;\; | \\ -NH-CH-(CH_2)_2-COR^{12} \end{array} \quad ;$$

$R^{11}$ représente le reste

$$\begin{array}{l} -CH-NHR^{14} \\ \;\;| \\ \;\;R^{13} \end{array} \quad ;$$

$R^{12}$ représente un hydroxy ou un alcoxy en $C_1$-$C_2$ ;
$R^{13}$ représente un groupe alkyle en $C_1$-$C_4$ ou benzyle ;
$R^{14}$ représente l'hydrogène ou un groupe benzyloxycarbonyle ;

ou une de leurs formes tautomères ou un de leurs sels pharmacologiquement acceptables.